(19) Europäisches Patentamt
European Patent Office
Office européen des brevets

(11) Veröffentlichungsnummer: **0 055 429**
**B1**

(12) # EUROPÄISCHE PATENTSCHRIFT

(45) Veröffentlichungstag der Patentschrift: **14.03.84**

(51) Int. Cl.³: **A 01 N 47/34,** C 07 C 145/04 // C07C145/02

(21) Anmeldenummer: **81110567.5**

(22) Anmeldetag: **18.12.81**

(54) **N-Sulfenylierte Allophanate, Verfahren zu ihrer Herstellung und ihre Verwendung als Fungizide.**

(30) Priorität: **30.12.80 DE 3049440**

(43) Veröffentlichungstag der Anmeldung: **07.07.82 Patentblatt 82/27**

(45) Bekanntmachung des Hinweises auf die Patenterteilung: **14.03.84 Patentblatt 84/11**

(84) Benannte Vertragsstaaten: **AT BE CH DE FR GB IT LI NL SE**

(56) Entgegenhaltungen: **CHEMISCHE BERICHTE, Nr. 10, 99. Jahrgang, 1966 H. PLIENINGER und D. WILD: "Reaktionen des 2-Äthoxy-indols und Oxindols" Seiten 3103-3107**

(73) Patentinhaber: **BAYER AG, Konzernverwaltung RP Patentabteilung, D-5090 Leverkusen 1 Bayerwerk (DE)**

(72) Erfinder: **Kühle, Engelbert, Dr., von-Bodelschwingh-Strasse 42, D-5060 Bergisch-Gladbach 2 (DE)**
Erfinder: **Hagemann, Hermann, Dr., Kandinskystrasse 52, D-5090 Leverkusen 1 (DE)**
Erfinder: **Paul, Volker, Dr., Ahornstrasse 5, D-5650 Solingen 1 (DE)**
Erfinder: **Brandes, Wilhelm, Dr., Eichendorffstrasse 3, D-5653 Leichlingen 1 (DE)**

**EP 0 055 429 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## N-Sulfenylierte Allophanate, Verfahren zu ihrer Herstellung und ihre Verwendung als Fungizide

Die Erfindung betrifft neue N-sulfenylierte Allophanate, Verfahren zu ihrer Herstellung sowie ihre Verwendung als Fungizide.

Es ist bereits lange bekannt, daß N-Trihalogenmethylthio-Verbindungen als Fungizide in der Landwirtschaft Verwendung finden können. So werden z. B. N-(Trichlormethylthio)-tetrahydrophthalimid bzw. N,N-Dimethyl-N'-phenyl-N'-(fluordichlormethylthio)-sulfamid im Obst- und Weinbau zur Bekämpfung von Pilzkrankheiten praktisch eingesetzt (vgl. DE-PS 887 506 und Angew. Chem. 76, 807 [1964]). In tropischen Kulturen, z. B. in Reis, ist ihre Wirkung jedoch nicht ausreichend. Dasselbe gilt für die ebenfalls schon lange bekannten Zinksalze von Alkylen-bis-dithiocarbaminsäuren, die ansonsten als Fungizide weltweite Bedeutung besitzen (vgl. z. B. R. Wegler, »Chemie der Pflanzenschutz- und Schädlingsbekämpfungsmittel«, Band 2, Seite 65, Springer-Verlag, Berlin/Heidelberg/New York [1970]).

Weiterhin sind Sulfenylallophanate, wie N-(Trifluormethansulfenyl)-allophansäuremethylester bzw. -benzylester, bekannt (vgl. Chemische Berichte 99. Jahrgang Nr. 10 [1966] Seiten 3104–3105). Eine Wirkung dieser Verbindungen wird nicht angesehen.

Es wurden nun als neue Stoffe die N-sulfenylierten Allophanate der allgemeinen Formel

$$R^1-S-\underset{\displaystyle R^2}{\underset{|}{N}}-\underset{\displaystyle O}{\underset{\|}{C}}-NH-\underset{\displaystyle O}{\underset{\|}{C}}-X-R^3 \qquad (I)$$

in welcher

$R^1$ für einen Trihalogenmethyl-Rest steht,

$R^2$ und $R^3$ für einen jeweils gegebenenfalls substituierten aliphatischen, cycloaliphatischen, araliphatischen oder aromatischen Rest stehen und

X für Sauerstoff oder Schwefel steht,

gefunden.

Die neuen Verbindungen der allgemeinen Formel (I) werden erhalten, indem man

a) ein Isocyanat der Formel

$$R^1-S-\underset{\displaystyle R^2}{\underset{|}{N}}-CO-NCO \qquad (II)$$

in welcher

$R^1$ und $R^2$ die oben angegebene Bedeutung haben,

mit einer Hydroxy- oder Mercapto-Verbindung der Formel

$$H-X-R^3 \qquad (III)$$

in welcher

$R^3$ und X die oben angegebene Bedeutung haben,

gegebenenfalls in Gegenwart eines Verdünnungsmittels und/oder eines Katalysators umsetzt, oder

b) ein Sulfensäureamid der Formel

$$R^1-S-\underset{\displaystyle R^2}{\underset{|}{NH}} \qquad (IV)$$

in welcher

$R^1$ und $R^2$ die oben angegebene Bedeutung haben,

mit einem Acylisocyanat der Formel

$$OCN-CO-X-R^3 \qquad (V)$$

in welcher

$R^3$ und X die oben angegebene Bedeutung haben,

gegebenenfalls in Gegenwart eines Verdünnungsmittels und/oder eines Katalysators umsetzt.

Die neuen N-sulfenylierten Allophanate der Formel (I) weisen starke fungizide Eigenschaften auf. Überraschenderweise zeigen die erfindungsgemäßen N-sulfenylierten Allophanate eine erheblich höhere fungizide Wirkung, insbesondere in Reiskulturen, als aus dem Stande der Technik bekannte Verbindungen. Die erfindungsgemäßen Stoffe stellen somit eine Bereicherung der Technik dar.

Die erfindungsgemäßen N-sulfenylierten Allophanate sind durch die Formel (I) allgemein definiert. In dieser Formel steht $R^1$ vorzugsweise für Trichlormethyl oder Fluordichlormethyl; $R^2$ steht vorzugsweise für einen gegebenenfalls halogenierten gesättigten oder ungesättigten aliphatischen Rest mit bis zu 6 Kohlenstoffatomen, der gegebenenfalls durch Heteroatome, wie Sauerstoff oder Stickstoff, unterbrochen sein kann, für einen cycloaliphatischen Rest mit 5 bis 8 Kohlenstoffatomen, für einen araliphatischen Rest mit 7 bis 10 Kohlenstoffatomen, dessen aromatischer Teil gegebenenfalls durch Halogen, Cyano-, Nitro-, Trifluormethyl-, Alkyl und/oder Alkoxy-, Alkylthio-Gruppen mit bis zu 4 Kohlenstoffatomen substituiert sein kann, und schließlich für einen aromatischen Rest mit 6 bis 10 Ring-Kohlenstoffatomen, der gegebenenfalls durch Halogen, Nitro-, Cyano-, Trifluormethyl-, Alkyl-, Alkylthio- und/oder Alkoxy-Gruppen mit jeweils bis zu 4 Kohlenstoffatomen substituiert sein kann; $R^3$ steht vorzugsweise für einen gegebenenfalls halogenierten gesättigten oder ungesättigtten aliphatischen Rest mit bis zu 8 Kohlenstoffatomen, der durch Heteroatome wie Sauerstoff, Schwefel und/oder Stickstoff unterbrochen sein kann, für einen gegebenenfalls durch Alkyl mit bis zu 4 Kohlenstoffatomen substituierten cycloaliphatischen Rest mit 5 bis 7 Kohlenstoffatomen, für einen araliphatischen Rest mit 7 bis 10 Kohlenstoffatomen, dessen aromatischer Teil substituiert sein kann durch Halogen, Cyano-, Nitro-, Trifluormethyl-, Alkyl-, Alkylthio und/oder Alkoxy-Gruppen mit bis zu 4 Kohlenstoffatomen, und schließlich für einen aromatischen Rest mit 6 bis 10 Ring-Kohlenstoffatomen, der gegebenenfalls durch Halogen, Nitro-, Cyano-, Trifluormethyl-, Alkyl-, Alkylthio und Alkoxy-Gruppen mit jeweils bis zu 4 Kohlenstoffatomen substituiert sein kann.

Ganz besonders bevorzugt sind diejenigen Verbindungen der allgemeinen Formel (I), in welchen

$R^1$ für Trichlormethyl und Fluordichlormethyl steht,
$R^2$ für Alkyl-, Alkenyl- und Halogenalkyl-Gruppen mit jeweils bis zu 4 Kohlenstoffatomen, für Cyclopentyl oder Cyclohexyl steht, und für Benzyl und für Phenyl steht, wobei die beiden letztgenannten Reste durch Halogen, Nitro-, Methyl- und/oder Trifluormethyl-Gruppen substituiert sein können, und
$R^3$ für Alkyl-, Alkenyl- und Halogenalkyl-Gruppen mit jeweils bis zu 4 Kohlenstoffatomen, für Cyclopentyl und für Cyclohexyl steht, und für Benzyl und für Phenyl steht, wobei die beiden letztgenannten Reste durch Halogen, Nitro-, Methyl-, Methoxy-, Methylthio-, Cyano- und/oder Trifluormethyl-Gruppen substituiert sein können, und
X für Sauerstoff oder Schwefel steht.

Verwendet man zur Herstellung der Verbindungen der Formel (I) gemäß Verfahrensvariante a) N-(Fluordichlormethylthio)-N-(methyl)-carbamoylisocyanat und Phenol bzw. gemäß Verfahrensvariante b) N-(Fluordichlormethylthio)-N-methylamid und Phenoxy-carbonylisocyanat als Ausgangskomponenten, so kann der Reaktionsverlauf durch das nachfolgende Reaktionsschema wiedergegeben werden:

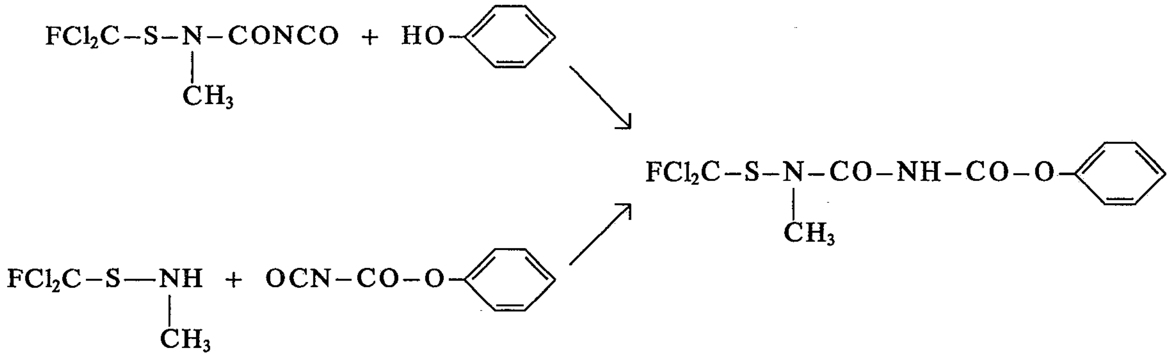

Die als Ausgangsstoffe für die Verfahrensvariante a) zu verwendenden Isocyanate sind durch die Formel (II) allgemein definiert. In dieser Formel stehen $R^1$ und $R^2$ vorzugsweise für diejenigen Reste, die bereits in Zusammenhang mit der Beschreibung der erfinungsgemäßen Stoffe der Formel (I) vorzugsweise für diese Substituenten genannten wurden.

Die Isocyanate der Formel (II) sind bisher noch nicht beschrieben (sie sind Gegenstand der EP-A-55 430. Sie lassen sich herstellen, indem man Trihalogenmethansulfenamide der allgemeinen Formel

$$\begin{array}{l} R^1\text{—S—NH} \\ \qquad\quad\ | \\ \qquad\quad R^2 \end{array} \qquad \text{(IV)}$$

in welcher

$R^1$ und $R^2$ die oben angegebene Bedeutung besitzen,

mit Chlorcarbonylisocyanat der Formel

$$Cl-CO-NCO \qquad \text{(VI)}$$

gegebenenfalls in Gegenwart eines Verdünnungsmittels im Temperaturbreich zwischen 0 und 150°C umsetzt (vgl. auch die Herstellungsbeispiele).

Geeignete Isocyanate der Formel (II) sind die Carbonylisocyanate des N-(Trichlormethylsulfenyl)-, N-Fluordichlormethylsulfenyl)- und N-(Trifluormethylsulfenyl)-methylamins, -isopropylamins, -allylamins, -2-methoxyethylamins, -tert.-butylamins, -cyclopentylamins, -cyclohexylamins, -benzylamins, -4-chlorbenzylamins, -phenethylamins, -anilins- -3-trifluormethylanilins, -3,4-dichloranilins, -3-anisidins und -3-toluidins.

Die für die Verfahrensvariante a) weiterhin benötigten Hydroxy- oder Mercapto-Verbindungen sind durch die Formel (III) definiert. In dieser Formel stehen X und $R^3$ vorzugsweise für diejenigen Reste, die bereits in Zusammenhang mit der Beschreibung der Verbindungen der Formel (I) vorzugsweise für diese Substituenten genannt wurden.

Als Verbindungen der Formel (III) eignen sich Methanol, Ethanol und Isopropanol, ferner Allyl-, Isobutyl-, Cyclohexyl-, 4-Methylcyclohexyl-, Benzyl-, Phenethyl- und 4-Chlorbenzyl-alkohol, sodann Phenol, 4-Chlorphenol, 4-Kresol, Hydrochinon-monomethylether, Methylmercaptan, Butylmercaptan, Thiophenol und 4-Methylthiophenol.

Die Verbindungen der Formel (III) sind allgemein bekannte Verbindungen der organischen Chemie.

Die für die Verfahrensvariante b) als Ausgangsstoffe zu verwendenden Sulfensäureamide sind durch die Formel (IV) allgemein definiert. In dieser Formel stehen $R^1$ und $R^2$ vorzugsweise für diejenigen Reste, die bereits bei Beschreibung der Verbindungen der Formel (I) vorzugsweise für diese Substituenten genannt wurden. Die Verbindungen sind durch Umsetzung von den entsprechenden primären Aminen mit Trihalogenmethansulfensäurechlorid, z. B. in Toluol als Lösungsmittel, im Temperaturbereich zwischen +20 und 30°C erhältlich (vgl. Chem. Abstr. 60, 5519 [1964]).

Die weiterhin für die Variante b) als Ausgangsstoffe benötigten Acylisocyanate sind durch die Formel (V) definiert. In dieser Formel stehen X und $R^3$ vorzugsweise für diejenigen Reste, die bereits im Zusammenhang mit der Beschreibung der Verbindungen der Formel (I) vorzugsweise für diese Reste genannt wurden. Die Acylisocyanate der Formel (V) sind bekannt oder können in bekannter Weise aus Alkoholen, Phenolen oder den entsprechenden Thiol-Verbindungen und Chlorcarbonylisocyanat hergestellt werden (vgl. Angew. Chem. 89, 789 [1977]). Dabei können als Alkohole und Phenole bzw. den entsprechenden Thiol-Verbindungen die weiter oben aufgeführten Verbindungen der Formel (III) Verwendung finden.

Für die erfindungsgemäßen Umsetzungen gemäß Verfahrensvarianten a) und b) kommen als Verdünnungsmittel inerte organische Lösungsmittel in Frage. Hierzu gehören vorzugsweise Kohlenwasserstoffe, wie Benzol oder Toluol, Ether, wie Diethylether oder Tetrahydrofuran, Chlorkohlenwasserstoffe, wie Chloroform oder Tetrachlorkohlenstoff.

Die Umsetzungen gemäß Varianten a) und b) können zwecks schnellerem und vollständigem Reaktionsablauf in Gegenwart eines basischen Katalysators, wie z. B. eines Amins, durchgeführt werden. Vorzugsweise verwendet man geringe Mengen Triethylamin.

Die Reaktionstemperaturen können bei den Verfahren a) und b) in einem größeren Bereich variiert werden. Im allgemeinen arbeitet man zwischen −10 und +100°C, vorzugsweise zwischen +10 und 50°C.

Bei der Durchführung der erfindungsgemäßen Verfahren a) bzw. b) setzt man auf 1 Mol der Verbindungen der Formeln (II) bzw. (IV) vorzugsweise 1 Mol der Verbindungen der Formeln (III) bzw. (V) ein. Zweckmäßigerweise werden die Isocyanate der Formel (II) bzw. die Acylisocyanate der Formel (V) vorgelegt und die Verbindungen der Formel (III) (bei Verfahren a)) bzw. der Formel (IV) (bei

Verfahren b)) werden unter Rühren zugefügt. Die Isolierung der Endprodukte erfolgt in allgemein üblicher Weise; die Endprodukte stellen zumeist nicht destillierbare Öle oder Kristallisate dar.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Pyricularia oryzae, dem Erreger der Brusone-Krankheit, und Pellicularia sasakii, dem Erreger der Blattscheidendürre an Reis, eingesetzt werden. Gute Erfolge werden auch gegen Grauschimmel (Botrytis cinerea) und gegen Apfelschorf (Venturia inaequalis) erzielt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel könnnen z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulte aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendunsformen in Mischung mit andren bekannten Wirkstoffen vorliegen, wie Fungiziden, Bakteriziden, Insektiziden, Akazifziden, Nematiziden, Herbiziden, Schutzstoffen gegen Vogelfraß, Wuchsstoffen, Pflanzennährstoffen und Bodensturkturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je

Kilogramm Saatgut, vorzugsweise 0,01 bis 10 g, benötigt.

Bei Behandung des Bodens sind Wirkstoffkonzentrationen von 0,00001 bis 0,1 Gew.-%, vorzugsweise von 0,0001 bi 0,02%, am Wirkungsort erforderlich.

## Herstellungsbeispiele

### Beispiel 1

(Verfahren b))

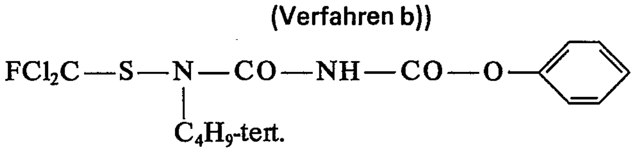

Zu 48,8 g (0,3 Mol) Phenoxycarbonylisocyanat, gelöst in 150 ml trockenem Toluol, tropft man bei Raumtemperatur 62 g (0,3 Mol) des aus Fluordichlormethansulfenylchlorid und tert.-Butylamin hergestellten (Fluordichlormethansulfenyl)N-tert.-butylamids ($Kp_{13}$ 60 – 65° C), gelöst in 30 ml Toluol. Hierbei steigt die Temperatur bis 44° C an. Nach dem Abkühlen kristallisieren 39 g N-(Fluordichlormethansulfenyl)-N-(tert.-butyl)-allophansäure-phenylester vom Fp. 101 – 102° C aus. Aus der Mutterlauge können durch Zusatz von Petrolether weitere 34 g des Reaktionsproduktes gewonnen werden. Man erhält somit insgesamt 73 g der genannten Verbindung, die Ausbeute beträgt 66% der Theorie.

### Beispiel 2

(Verfahren b))

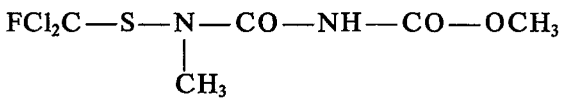

Zu einer Lösung von 5 g (0,05 Mol) Methoxycarbonylisocyanat in 100 ml trockenem Toluol tropft man 8,2 g (0,05 Mol) Fluordichlormethansulfenyl-methylamid. Hierbei steigt die Temperatur bis etwa 35° C an. Nach Abklingen der Reaktion wird im Vakuum eingeengt. Der Rückstand (12 g) wird aus Ligroin umkristallisiert. Man erhält hierbei 10 g N-(Fluordichlormethansulfenyl)-N-(methyl)-allophansäuremethylester vom Fp. 74 – 76° C, das sind 76% der Theorie.

### Beispiel 3

(Verfahren a))

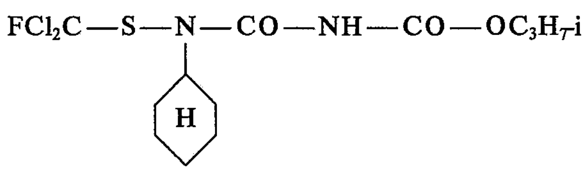

Zu der Lösung von 15 g (0,05 Mol) N-(Fluordichlormethansulfenyl)-N-(cyclohexyl)-amido-carbonylisocyanat ($Kp_{11}$ 158 – 160° C) in 100 ml Toluol tropft man 10 ml Isopropanol, dem einige Tropfen Triethylamin beigemischt sind, zu. Hierbei steigt die Temperatur auf etwa 33° C an. Man rührt eine Zeitlang nach und engt im Vakuum ein. Man erhält das Reaktionsprodukt in Form eines Öls vom $n_D^{20}$ = 1,4975. Die Ausbeute ist quantitativ.

### Herstellung des Vorproduktes

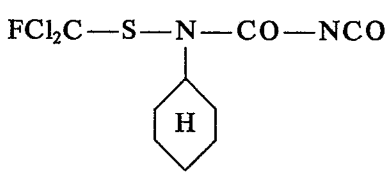

In die Lösung von 44 g (0,4 Mol) Chlorcarbonylisocyanat in 150 ml Chlorbenzol tropft man unter

Eiskühlung bei +10 bis 20°C eine Lösung von 45 g (0,2 Mol) Fluordichlormethan-sulfenyl-N-(cyclohexyl)-amid ($Kp_{0,1}$ 68 bis 70°C) in 50 ml Chlorbenzol ein. Anschließend erhitzt man die Reaktionslösung allmählich zum Sieden, wobei ab etwa 70–80°C Chlorwasserstoff abgespalten wird. Man erhält 1 Stunde bei Rückflußtemperatur, engt die Lösung im Vakuum ein und destilliert den Rückstand. Man erhält 46 g N-(Fluordichlormethan-sulfenyl)-N-(cyclohexyl)-amido-carbonylisocyanat vom $Kp_{11}$ 158 bis 160°C. Die Ausbeute beträgt 76% der Theorie.

Das als Ausgangsprodukt benötigte Chlorcarbonylisocyanat ist aus der Literatur bekannt (vgl. Angew. Chem. 89, 789 [1977]) und ausführlich beschrieben. Es wird zunächst das Additionsprodukt aus Phosgen und Chlorcyan am Aktivkohle-Kontakt hergestellt und das Addukt hernach mit Methansulfonsäure verseift, wobei man Chlorcarbonylisocyanat in 90%iger Ausbeute erhält.

In entsprechender Weise, wie in den Beispielen 1 bis 3 angegeben, erhält man die folgenden Verbindungen der allgemeinen Formel

$$R^1—S—\underset{\displaystyle R^2}{\underset{|}{N}}—CO—NH—CO—X—R^3 \quad (I)$$

| Beispiel Nr. | $R^1$ | $R^2$ | $XR^3$ | Fp. (°C) oder Brechungsindex |
|---|---|---|---|---|
| 4 | $FCl_2C$ | $C_3H_7$-i | 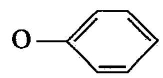 | $n_D^{20} = 1,5379$ |
| 5 | $FCl_2C$ | 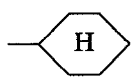 | 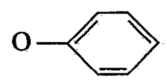 | $n_D^{20} = 1,5389$ |
| 6 | $Cl_3C$ | 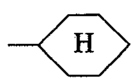 | 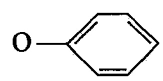 | $n_D^{20} = 1,5570$ |
| 7 | $FCl_2C$ | 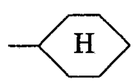 | 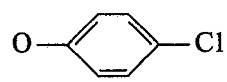 | 129–130 |
| 8 | $FCl_2C$ | $CH_3$ | 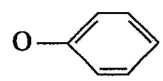 | 100–102 |
| 9 | $FCl_2C$ | $CH_3$ | $S—C_4H_9$-n | 69–70 |
| 10 | $FCl_2C$ | $C_3H_7$-i | $O—CH_3$ | $n_D^{20} = 1,4983$ |
| 11 | $FCl_2C$ | 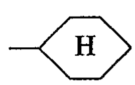 | $S—C_3H_7$-n | 91–94 |
| 12 | $Cl_3C$ | $C_3H_7$-i | $S—C_3H_7$-n | 70–72 |
| 13 | $Cl_3C$ | $C_4H_9$-n | $S—C_3H_7$-n | $n_D^{20} = 1,5392$ |
| 14 | $FCl_2C$ | $CH_3$ | $O—CH_2—CF_3$ | 55–59 |
| 15 | $FCl_2C$ | $CH_3$ | 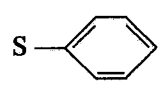 | 70–72 |
| 16 | $FCl_2C$ | $C_4H_9$-t | $O—C_3H_7$-i | |
| 17 | $FCl_2C$ | $CH_3$ | 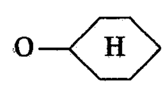 | 105–107 |

Fortsetzung

| Beispiel Nr. | $R^1$ | $R^2$ | $XR^3$ | Fp. (°C) oder Brechungsindex |
|---|---|---|---|---|
| 18 | $FCl_2C$ | $C_2H_5$ | 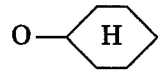 | 65 – 67 |
| 19 | $FCl_2C$ | $C_3H_7$-n | 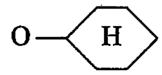 | $n_D^{20}$ = 1,5029 |
| 20 | $FCl_2C$ | $C_3H_7$-i | 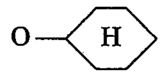 | 68 – 70 |
| 21 | $FCl_2C$ | $CH_2—CH=CH_2$ | 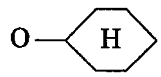 | 62 – 63 |
| 22 | $FCl_2C$ | $C_4H_9$-n | 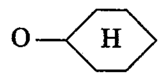 | $n_D^{20}$ = 1,5020 |
| 23 | $FCl_2C$ | $C_4H_9$-i | 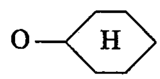 | 70 – 72 |
| 24 | $FCl_2C$ | $C_4H_9$-t | 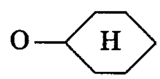 | $n_D^{20}$ = 1,5038 |
| 25 | $FCl_2C$ | $CH_3$ | 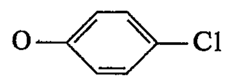 | 113 – 114 |
| 26 | $FCl_2C$ | $C_2H_5$ | 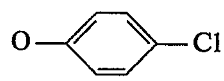 | 110 – 112 |
| 27 | $FCl_2C$ | $C_3H_7$-n | 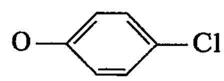 | 102 – 104 |
| 28 | $FCl_2C$ | $C_3H_7$-i | 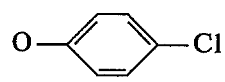 | 105 |
| 29 | $FCl_2C$ | $CH_2—CH=CH_2$ | 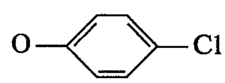 | 78 – 80 |
| 30 | $FCl_2C$ | $C_4H_9$-n | 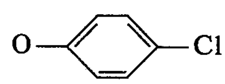 | 77 – 79 |
| 31 | $FCl_2C$ | $C_4H_9$-i | 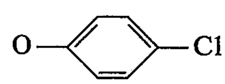 | 83 – 85 |
| 32 | $FCl_2C$ | $C_4H_9$-t | 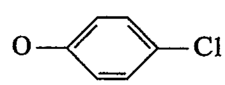 | 89 – 92 |
| 33 | $FCl_2C$ | $C_3H_7$-i | 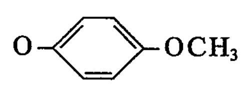 | $n_D^{20}$ = 1,5365 |
| 34 | $FCl_2C$ | $C_4H_9$-i | 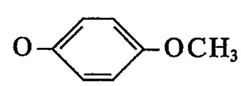 | 76 – 80 |
| 35 | $FCl_2C$ | $C_4H_9$-t | 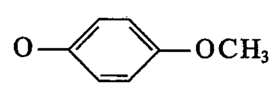 | 115 – 120 |

Fortsetzung

| Beispiel Nr. | $R^1$ | $R^2$ | $XR^3$ | Fp. (°C) oder Brechungsindex |
|---|---|---|---|---|
| 36 | $FCl_2C$ | $C_3H_7$-i | 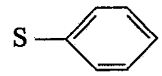 | 75 – 78 |
| 37 | $FCl_2C$ | $C_4H_9$-i | 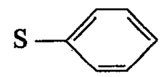 | 89 – 90 |
| 38 | $FCl_2C$ | $C_4H_9$-t | 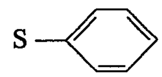 | 82 – 85 |
| 39 | $FCl_2C$ | $C_3H_7$-i | 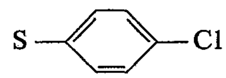 | 98 – 99 |
| 40 | $FCl_2C$ | $C_4H_9$-i | 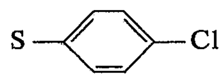 | 77 – 80 |
| 41 | $FCl_2C$ | $C_4H_9$-t | 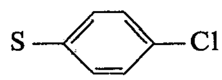 | 109 – 112 |
| 42 | $FCl_2C$ | 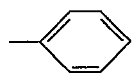 | $OCH_3$ | $n_D^{20} = 1,5618$ |

## Verwendungsbeispiele

In den nachfolgenden Verwendungsbeispielen wurde die nachstehend angegebene Verbindung als Vergleichssubstanz herangezogen:

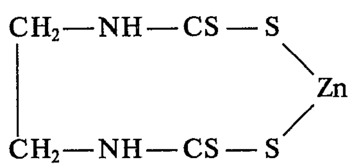

(A)

### Beispiel A

Pyricularia-Test (Reis) / protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25° C aufgestellt.

4 Tage nach der Inokulation erfolgt die Auswertung des Krankheitsbefalls.

Eine deutliche Überlegenheit in der Wirksamkeit gegenüber dem Stand der Technik zeigen bei diesem Test z. B. die Verbindungen gemäß folgender Herstellungsbeispiele: 42, 9 und 5.

## Beispiel B

### Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und in einem Gewächshaus bei 25°C und 100% relativer Luftfeuchtigkeit aufgestellt.

5–8 Tage nach der Inokulation erfolgt die Auswertung des Krankheitsbefalles.

Eine deutliche Überlegenheit in der Wirksamkeit gegenüber dem Stand der Technik zeigen bei diesem Test z. B. die Verbindungen gemäß folgender Herstellungsbeispiele: 42, 9 und 5.

## Beispiel C

### Botrytis-Test (Bohnen) / protektiv

Lösungsmittel: 4,7 Gewichtsteile Aceton
Dispergiermittel: 0,3 Gewichtsteile Alkyl-aryl-polyglykol-ether
Wasser: 95 Gewichtsteile

Man vermischt die für die gewünschte Wirkstoffkonzentration in der Spritzflüssigkeit nötige Wikstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man Pflanzen von Phaseolus vulgaris im 2-Blattstadium bis zur Tropfnässe. Nach 24 Stunden werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern bonitiert.

Die erhaltenen Boniturwerte werden auf Prozent Befall umgerechnet. 0% bedeutet keinen Befall, 100% bedeutet, daß der Befallsfleck vollständig ausgebildet ist.

Eine deutliche Überlegenheit in der Wirksamkeit gegenüber dem Stand der Technik zeigen bei diesem Test z. B. die Verbindungen gemäß folgender Herstellungsbeispiele 9.

**Patentansprüche**

1. N-Sulfenylierte Allophanate der Formel

$$R^1-S-\underset{\displaystyle R^2}{\underset{|}{N}}-CO-NH-CO-X-R^3 \qquad (I)$$

in welcher

- $R^1$ für einen Trihalogenmethyl-Rest steht,
- $R^2$ für einen jeweils gegebenenfalls substituierten aliphatischen, cycloaliphatischen, araliphatischen oder aromatischen Rest steht,
- $R^3$ für einen jeweils gegebenenfalls substituierten aliphatischen, cycloaliphatischen, araliphatischen oder aromatischen Rest steht, und
- X für Sauerstoff oder Schwefel steht.

2. Verbindungen der Formel (I) in Anspruch 1, wobei

- $R^1$ für Trichlormethyl und Fluordichlormethyl steht,
- $R^2$ für Alkyl-, Alkenyl- und Halogenalkyl-Gruppen mit jeweils bis zu 4 Kohlenstoffatomen, für Cyclopentyl oder Cyclohexyl steht, und für Benzyl und für Phenyl steht, wobei die beiden letztgenannten Reste durch Halogen, Nitro-, Methyl- und/oder Trifluormethyl-Gruppen substituiert sein können, und

$R^3$ für Alkyl-, Alkenyl- und Halogenalkyl-Gruppen mit jeweils bis zu 4 Kohlenstoffatomen, für Cyclopentyl und für Cyclohexyl steht, und für Benzyl und für Phenyl steht, wobei die beiden letztgenannten Reste durch Halogen, Nitro-, Methyl-, Methoxy-, Methylthio-, Cyano- und/oder Trifluormethyl-Gruppen substituiert sein können, und

X für Sauerstoff oder Schwefel steht.

3. Verfahren zur Herstellung von N-sulfenylierten Allophanaten der Formel

$$R^1—S—\underset{\displaystyle R^2}{\underset{|}{N}}—CO—NH—CO—X—R^3 \qquad (I)$$

in welcher

$R^1$ für einen Trihalogenmethyl-Rest steht,

$R^2$ für einen jeweils gegebenenfalls substituierten aliphatischen, cycloaliphatischen, araliphatischen oder aromatischen Rest steht,

$R^3$ für einen jeweils gegebenenfalls substituierten aliphatischen, cycloaliphatischen, araliphatischen oder aromatischen Rest steht, und

X für Sauerstoff oder Schwefel steht,

dadurch gekennzeichnet, daß man

a) ein Isocyanat der Formel

$$R^1—S—\underset{\displaystyle R^2}{\underset{|}{N}}—CO—NCO \qquad (II)$$

in welcher

$R^1$ und $R^2$ die oben angegebene Bedeutung haben,

mit einer Hydroxy- oder Mercapto-Verbindung der Formel

$$H-X-R^3 \qquad (III)$$

in welcher

$R^3$ und X die oben angegebene Bedeutung haben,

gegebenenfalls in Gegenwart eines Verdünnungsmittels und/oder eines Katalysators umsetzt, oder

b) ein Sulfensäureamid der Formel

$$R^1—S—\underset{\displaystyle R^2}{\underset{|}{NH}} \qquad (IV)$$

in welcher

$R^1$ und $R^2$ die oben angegebene Bedeutung haben,

mit einem Acylisocyanat der Formel

$$OCN-CO-X-R^3 \qquad (V)$$

in welcher

$R^3$ und X die oben angegebene Bedeutung haben,

gegebenenfalls in Gegenwart eines Verdünnungsmittels und/oder eines Katalysators umsetzt.

4. Fungizide Mittel, gekennzeichnet durch einen Gehalt an mindestens einem N-sulfenylierten Allophanat der Formel (I) in Ansprüchen 1 und 3.

5. Verfahren zur Bekämpfung von Pilzen, dadurch gekennzeichnet, daß man N-sulfenylierte Allophanate der Formel (I) in Ansprüchen 1 und 3 auf Pilze oder ihren Lebensraum einwirken läßt.

6. Verwendung von N-sulfenylierten Allophanaten der Formel (I) in Ansprüchen 1 und 3 zur Bekämpfung von Pilzen.

7. Verfahren zur Herstellung von fungiziden Mitteln, dadurch gekennzeichnet, daß man N-sulfenylierte Allophanate der Formel (I) in Ansprüchen 1 und 3 mit Streckmitteln und/oder oberflächenaktiven Mitteln vermischt.

## Claims

1. N-Sulphenylated allophanates of the formula

$$\begin{array}{l} R^1-S-\underset{\displaystyle R^2}{\underset{|}{N}}-CO-NH-CO-X-R^3 \end{array} \qquad (I)$$

in which

$R^1$ represents a trihalogenomethyl radical,
$R^2$ represents an aliphatic, cycloaliphatic, araliphatic or aromatic radical which is in each case optionally substituted,
$R^3$ represents an aliphatic, cycloaliphatic, araliphatic or aromatic radical which is in each case optionally substituted and
X represents oxygen or sulphur.

2. Compounds of the formula (I) in Claim 1, wherein

$R^1$ represents trichloromethyl or fluorodichloromethyl,
$R^2$ represents an alkyl, alkenyl or halogenoalkyl group with in each case up to 4 carbon atoms, cyclopentyl or cyclohexyl, or benzyl or phenyl, it being possible for the two latter radicals to be substituted by halogen, nitro groups, methyl groups and/or trifluoromethyl groups,
$R^3$ represents an alkyl, alkenyl or halogenoalkyl group with in each case up to 4 carbon atoms, cyclopentyl or cyclohexyl, or benzyl or phenyl, it being possible for the two latter radicals to be substituted by halogen, nitro groups, methyl groups, methoxy groups, methylthio groups, cyano groups and/or trifluoromethyl groups, and
X represents oxygen or sulphur.

3. Process for the preparation of N-sulphenylated allophanates of the formula

$$R^1-S-\underset{\displaystyle R^2}{\underset{|}{N}}-CO-NH-CO-X-R^3 \qquad (I)$$

in which

$R^1$ represents a trihalogenomethyl radical,
$R^2$ represents an aliphatic, cycloaliphatic, araliphatic or aromatic radical which is in each case optionally substituted,
$R^3$ represents an aliphatic, cycloaliphatic, araliphatic or aromatic radical which is in each case optionally substituted and
X represents oxygen or sulphur,

characterised in that

a) an isocyanate of the formula

$$R^1-S-\underset{\displaystyle R^2}{\underset{|}{N}}-CO-NCO \qquad (II)$$

in which

$R^1$ and $R^2$ have the abovementioned meaning, is reacted with a hydroxy or mercapto compound of the formula

$H-X-R^3$ (III)

in which

$R^3$ and X have the abovementioned meaning,

if appropriate in the presence of a diluent and/or of a catalyst, or

b) a sulphenic acid amide of the formula

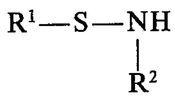 (IV)

in which

$R^1$ and $R^2$ have the abovementioned meaning, is reacted with an acyl isocyanate of the formula

$OCN-CO-X-R^3$ (V)

in which

$R^3$ and X have the abovementioned meaning,

if appropriate in the presence of a diluent and/or of a catalyst.

4. Fungicidal agents, characterised in that they contain at least one N-sulphenylated allophanate of the formula (I) in Claims 1 and 3.

5. Process for combating fungi, characterised in that N-sulphenylated allophanates of the formula (I) in Claims 1 and 3 are allowed to act on fungi or their environment.

6. Use of N-sulphenylated allophanates of the formula (I) in Claims 1 and 3 for combating fungi.

7. Process for the preparation of fungicidal agents, characterised in that N-sulphenylated allophanates of the formula (I) in Claims 1 and 3 are mixed with extenders and/or surface-active agents.

**Revendications**

1. Allophanates N-sulfénylés de formule

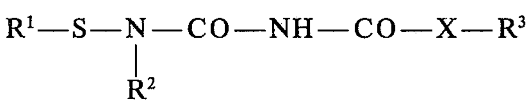 (I)

dans laquelle

$R^1$ est un reste trihalogénométhyle,

$R^2$ est un reste aliphatique, cycloaliphatique, araliphatique ou aromatique, chacun étant éventuellement substitué,

$R^3$ est un reste aliphatique, cycloaliphatique, araliphatique ou aromatique, chacun étant éventuellement substitué et

X représente l'oxygène ou le soufre.

2. Composés de formule (I) suivant la revendication 1, dans laquelle

$R^1$ est un groupe trichlorométhyle ou fluorodichlorométhyle,

$R^2$ représente des groupes alkyle, alcényle et halogénalkyle ayant chacun jusqu'à 4 atomes de carbone, le groupe cyclopentyle ou le groupe cyclohexyle, et les groupes benzyle et phényle, les deux groupes mentionnés en dernier lieu pouvant être substitués par un halogène et des groupes nitro, méthyle et/ou trifluorométhyle, et

$R^3$ représente des groupes alkyle, alcényle et halogénalkyle ayant chacun jusqu'à 4 atomes de carbone, le groupe cyclopentyle et le groupe cyclohexyle et les groupes benzyle et phényle, les deux restes mentionnés en dernier lieu pouvant être substitués par un halogène, des groupes nitro, méthyle, méthoxy, méthylthio, cyano et/ou trifluorométhyle, et

X représente l'oxygène ou le soufre.

3. Procédé de production d'allophanates N-sulfénylés de formule

$$R^1—S—N(R^2)—CO—NH—CO—X—R^3 \quad (I)$$

dans laquelle

$R^1$ est un reste trihalogénométhyle,
$R^2$ est un reste aliphatique, cycloaliphatique, araliphatique ou aromatique, chacun étant éventuellement substitué,
$R^3$ est un reste aliphatique, cycloaliphatique, araliphatique ou aromatique, chacun étant éventuellement substitué, et
X représente l'oxygène ou le soufre,

caractérisé en ce que

a) on fait réagir un isocyanate de formule

$$R^1—S—N(R^2)—CO—NCO \quad (II)$$

dans laquelle

$R^1$ et $R^2$ ont la définition indiquée ci-dessus, avec un composé hydroxy ou mercapto de formule

$$H-X-R^3 \quad (III)$$

dans laquelle

$R^3$ et X ont la définition indiquée ci-dessus,

éventuellement en présence d'un diluant et/ou d'un catalyseur, ou

b) on fait réagir un amide d'acide sulfénique de formule

$$R^1—S—NH(R^2) \quad (IV)$$

dans laquelle

$R^1$ et $R^2$ ont la définition indiquée ci-dessus, avec un isocyanate d'acyle de formule

$$OCN-CO-X-R^3 \quad (V)$$

dans laquelle

$R^3$ et X ont la définition indiquée ci-dessus,

éventuellement en présence d'un diluant et/ou d'un catalyseur.

4. Compositions fongicides, caractérisées par une teneur en au moins un allophanate N-sulfénylé de formule (I) suivant les revendications 1 et 3.

5. Procédé pour combattre des champignons, caractérisé en ce qu'on fait agir des allophanates N-sulfénylés de formule (I) suivant les revendications 1 à 3 sur les champignons ou sur leur milieu.

6. Utilisation d'allophanates N-sulfénylés de formule (I) suivant les revendications 1 et 3 pour combattre des champignons.

7. Procédé de préparation de compositions fongicides, caractérisé en ce qu'on mélange des allophanates N-sulfénylés de formule (I) suivant les revendications 1 et 3 avec des diluants et/ou des agents tensio-actifs.